(19) Europäisches Patentamt
European Patent Office
Office européen des brevets

(11) Veröffentlichungsnummer: **0 243 774**
**A1**

(12) **EUROPÄISCHE PATENTANMELDUNG**

(21) Anmeldenummer: **87105460.7**

(51) Int. Cl.[4]: **C07F 7/08** , A01N 55/00

(22) Anmeldetag: **13.04.87**

(30) Priorität: **26.04.86 DE 3614249**

(43) Veröffentlichungstag der Anmeldung:
**04.11.87 Patentblatt 87/45**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Förster, Heinz, Dr.**
**Am Eckbusch 47**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**

(54) **Substituierte Chinoxalinyloxy-phenoxypropionsäure-Derivate.**

(57) Die Erfindung betrifft neue substituierte Chinoxalinyloxyphenoxypropionsäure-Derivate der Formel (I)

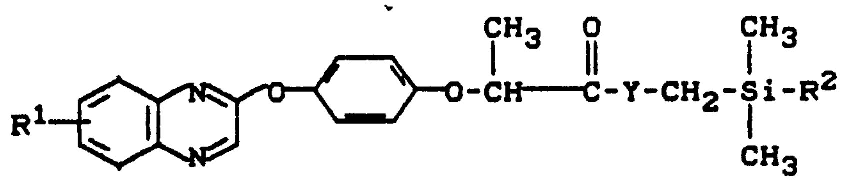

(I)

in welcher
Y für Sauerstoff, Schwefel oder die Gruppierung der Formel NR steht, wobei
R für Wasserstoff oder Alkyl mit 1 bis 6 Kohlenstoffatomen steht,
$R^1$ für Wasserstoff oder Halogen steht und
$R^2$ für Alkyl mit 1 bis 6 Kohlenstoffatomen steht,
mehrere Verfahren zu deren Herstellung und ihre Verwendung als Herbizide.

EP 0 243 774 A1

# Substituierte Chinoxalinyloxy-phenoxypropionsäure-Derivate

Die Erfindung betrifft neue substituierte Chinoxalinyloxyphenoxypropionsäure-Derivate, mehrere Verfahren zu deren Herstellung und ihre Verwendung als Herbizide.

Es ist bereits bekannt, daß zahlreiche Phenoxypropionsäure-Derivate herbizide Eigenschaften besitzen (vergl. EP-A 0 096 354). So kann z. B. das R-Enantiomere des 2-[4-(3,5-Dichlorpyridyl-2-oxy)-phenoxy]-propionsäure-(trimethylsilyl)-methylesters zur Bekämpfung von Unkraut eingesetzt werden. Die Wirkung dieser Stoffe ist gut, jedoch werden einige Unkräuter und Ungräser nicht immer voll erfaßt.

Es wurden nun neue substituierte Chinoxalinyloxy-phenoxypropionsäure-Derivate der Formel (I)

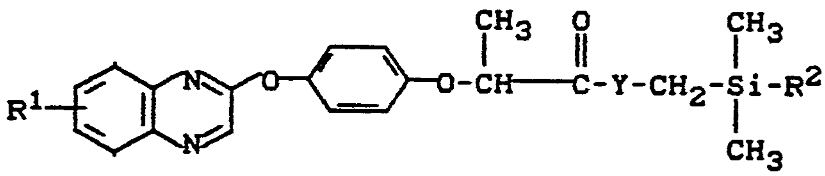

(I)

in welcher
Y für Sauerstoff, Schwefel oder die Gruppierung der Formel NR steht, wobei
R für Wasserstoff oder Alkyl mit 1 bis 6 Kohlenstoffatomen steht,
$R^1$ für Wasserstoff oder Halogen steht und
$R^2$ für Alkyl mit 1 bis 6 Kohlenstoffatomen steht,
gefunden.

Die substituierten Chinoxalinyloxy-phenoxypropionsäure-Derivate der Formel (I) enthalten mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Weiterhin wurde gefunden, daß man substituierte Chinoxalinyloxy-phenoxypropionsäure-Derivate der Formel (I) erhält, wenn man

a) Propionsäure-Derivate der Formel (II)

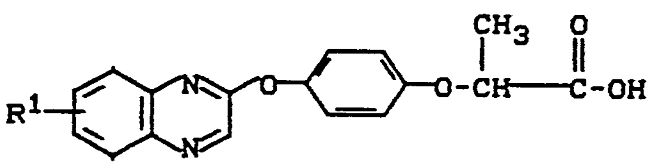

(II)

in welcher
$R^1$ die oben angegebene Bedeutung hat,
mit Silylchloriden der Formel (III)

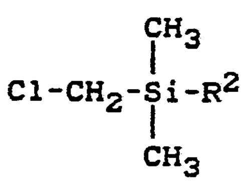

(III)

in welcher
$R^2$ die oben angegebene Bedeutung hat,
gegebenenfalls in Gegenwart eines Säurebindemittels sowie gegebenenfalls in Gegenwart eines Verdünnungsmittels umsetzt,
oder wenn man

b) Chinoxalinyloxy-phenoxypropionsäurechloride der Formel (IV)

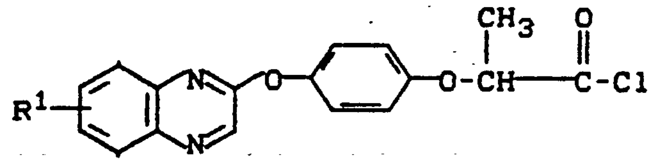

(IV)

in welcher
$R^1$ die oben angegebene Bedeutung hat,
mit Verbindungen der Formel (V)

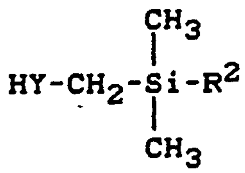

(V)

in welcher
$R^2$ und Y die oben angegebenen Bedeutungen haben,
gegebenenfalls in Gegenwart eines Säurebindemittels sowie gegebenenfalls in Gegenwart eines Verdünnungsmittels umsetzt, oder wenn man
c) Heterocyclen der Formel (VI)

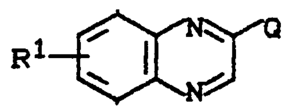

(VI)

in welcher
$R^1$ die oben angegebene Bedeutung hat und
Q für Halogen, Methylsulfonyl, Ethylsulfonyl oder Tosyl steht,
mit Hydrochinon-Derivaten der Formel (VII)

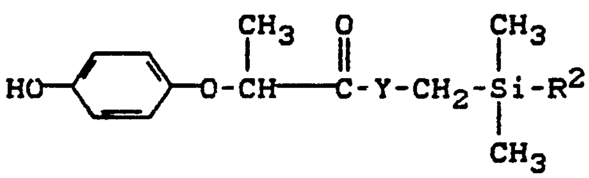

(VII)

in welcher
$R^2$ und Y die oben angegebenen Bedeutungen haben,
gegebenenfalls in Gegenwart eines Säurebindemittels sowie gegebenenfalls in Gegenwart eines Verdünnungsmittels umsetzt.

Schließlich wurde gefunden, das sich sie neuen substituierten Chinoxalinyloxy-phenoxypropionsäure-Derivate der Formel (I) durch eine hervorragende herbizide Wirksamkeit auszeichnen.

Überraschenderweise besitzen die erfindungsgemäßen substituierten Chinoxalinyloxy-phenoxypropionsäure-Derivate der Formel (I) wesentlich bessere herbizide Eigenschaften als der 2-[4-(3,5-Dichlorpyridiyl-2-oxy)-phenoxy]-propionsäure-(trimethylsilyl)-methylester, welcher konstitutionell ein ähnlicher vorbekannter Wirkstoff gleicher Wirkungsart ist.

Im Rahmen der Substituentendefinitionen steht Halogen für Fluor, Chlor, Brom und Iod und Alkyl steht für eine geradkettige oder verzweigte Kohlenwasserstoffkette.

Die erfindungsgemäßen substituierten Chinoxalinyloxy-phenoxypropionsäure-Derivate sind durch die Formel (I) allgemein definiert. In dieser Formel steht vorzugsweise
Y für Sauerstoff, Schwefel oder die Gruppierung der Formel NR, worin
R für Wasserstoff oder Alkyl mit 1 bis 4 Kohlenstoffatomen steht,
$R^1$ für Wasserstoff, Fluor, Chlor oder Brom und
$R^2$ für Alkyl mit 1 bis 4 Kohlenstoffatomen.

Besonders bevorzugt sind diejenigen Verbindungen der Formel (I), in denen
Y für Sauerstoff, Schwefel oder die Gruppierung der Formel NR steht, worin
R für Wasserstoff, Methyl oder Ethyl steht,
$R^1$ für Wasserstoff, Fluor oder Chlor steht und
$R^2$ für Methyl, Ethyl, n-Propyl oder n-Butyl steht.

Eine weitere Gruppe besonders bevorzugter erfindungsgemäßer Verbindungen sind weiterhin die substituierten Chinoxalinyloxy-phenoxypropionsäure-Derivate der Formel (I), in denen das asymmetrisch substituierte Kohlenstoffatom der Propionsäure-Einheit die R-Konfiguration aufweist und die Reste Y, $R^1$ und $R^2$ die oben angegebenen besonders bevorzugten Bedeutungen haben. Die betreffenden Stoffe lassen sich durch die Formel (Ia)

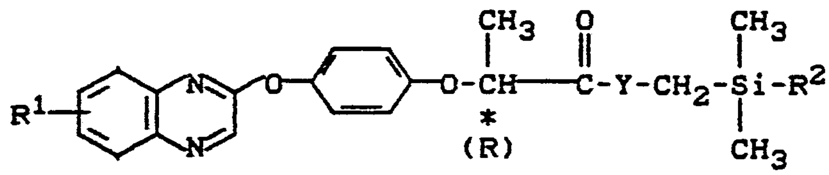

(Ia)

charakterisieren. In dieser Formel (Ia) ist das asymmetrisch substituierte Kohlenstoffatom durch ein (*) gekennzeichnet.

Als Beispiele für die erfindungsgemäßen Verbindungen der Formeln (I) bzw. (Ia) seien die in der folgenden Tabelle formelmäßig aufgeführten Verbindungen genannt:

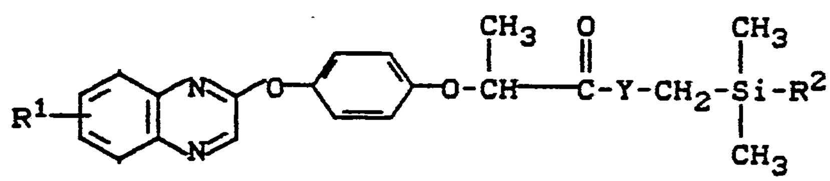

(I)

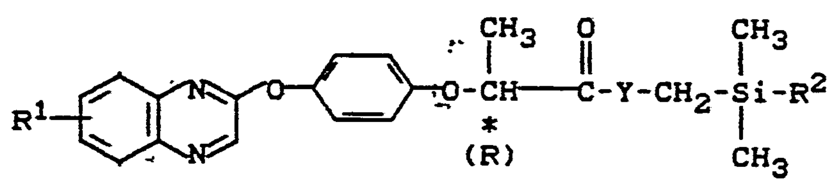

(Ia)

Tabelle 1

| $R^1$ | $R^2$ | Y |
|---|---|---|
| H | $-CH_3$ | S |
| 6-Cl | $-C_2H_5$ | O |
| 6-Cl | $-C_3H_7$-i | O |
| 6-Br | $-CH_3$ | O |
| 6-Cl | $-C_2H_5$ | S |
| 6-Br | $-CH_3$ | S |
| 6-F | $-CH_3$ | S |
| 5-Cl | $-CH_3$ | O |
| 5-Cl | $-CH_3$ | NH |
| 7-Cl | $-CH_3$ | S |
| 6-Cl | $-CH_3$ | $NCH_3$ |
| 8-Cl | $-CH_3$ | S |
| H | $-C_2H_5$ | O |
| 6-Cl | $-C_3H_7$-n | O |
| 6-Cl | $-C_4H_9$-n | O |
| 6-Cl | $-CH_3$ | S |
| 6-Cl | $-C_3H_7$-n | S |
| 6-F | $-CH_3$ | O |
| 6-F | $-CH_3$ | NH |
| 5-Cl | $-CH_3$ | S |
| 7-Cl | $-CH_3$ | O |
| 7-Cl | $-CH_3$ | NH |
| 8-Cl | $-CH_3$ | O |

Verwendet man 2-[4-(6-Chlor-chinoxalinyl-2-oxy)-phenoxy]-propionsäure und Chlormethyl-trimethylsilan als Ausgangsstoffe, so kann der Verlauf des erfindungsgemäßen Verfahrens (a) durch das folgende Formelschema wiedergegeben werden:

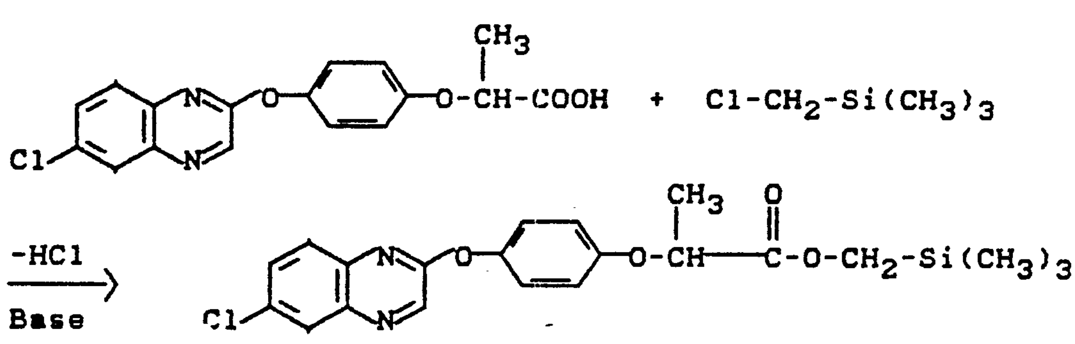

Verwendet man 2-[4-(6-Chlor-chinoxalinyl-2-oxy)-phenoxy]-propionsäurechlorid und (Dimethyl-ethylsilyl)-methylmercaptan als Ausgangsstoffe, so kann der Verlauf des erfindungsgemäßen Verfahrens (b) durch das folgende Formelschema wiedergegeben werden:

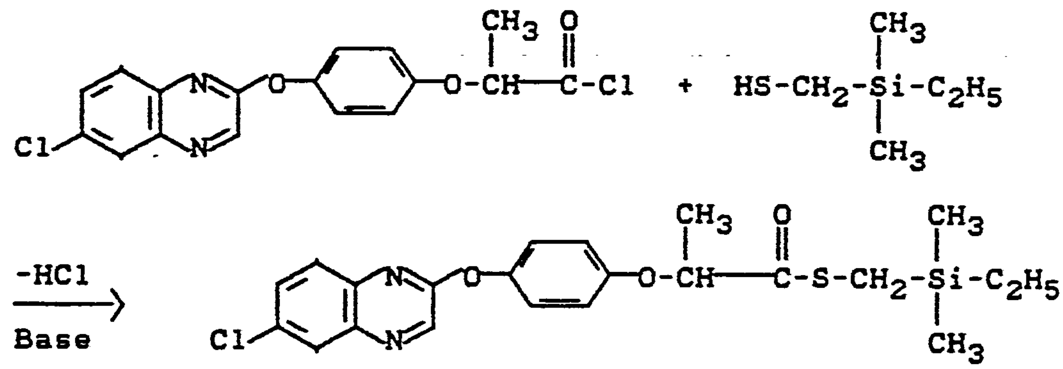


Verwendet man 2,6-Dichlor-chinoxalin und 2-(4-Hydroxyphenoxy)-propionsäure-(trimethylsilyl-methyl)-methylamid als Ausgangsstoffe so kann das erfindungsgemäße Verfahren (c) durch das folgende Formelschema wiedergegeben werden:

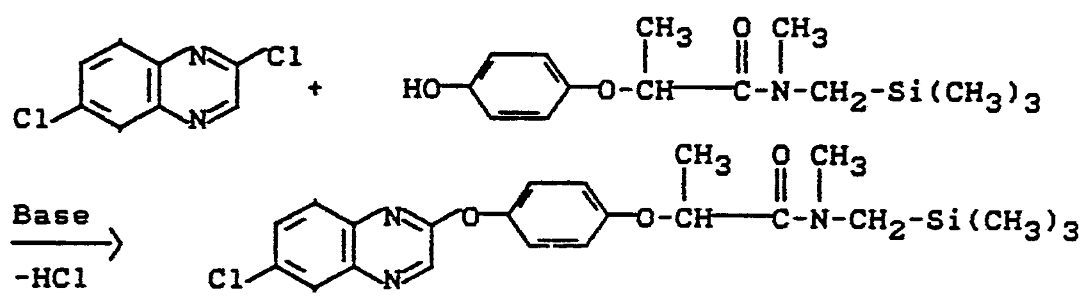


Die bei dem erfindungsgemäßen Verfahren (a) als Ausgangsstoffe benötigten Propionsäure-Derivate sind durch die Formel (II) definiert. In dieser Formel hat R' vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Propionsäure-Derivate der Formel (II) sind bekannt oder lassen sich nach bekannten Verfahren in einfacher Weise herstellen (vergl. z. B. DE-OS 30 04 770).

Zur Herstellung von R-und S-Enantiomeren der substituierten Chinoxalinyloxy-phenoxypropionsäure-Derivate der Formel (Ia) werden bei der Durchführung des erfindungsgemäßen Verfahrens (a) optisch aktive R-oder S-Propionsäure-Derivate der Formel (II) als Ausgangsstoffe benötigt. Auch diese optisch aktiven Propionsäure-Derivate der Formel (II) sind bekannt oder lassen sich nach prinzipiell bekannten Verfahren in einfacher Weise herstellen (vergl. z. B. DE-OS 30 04 770).

Man erhält R-bzw. S-Enantiomere der Propionsäure-Derivate der Formel (II)

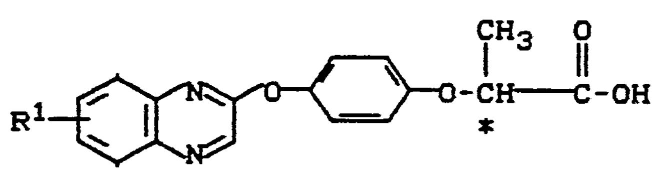
(II)

in welcher
R' die oben angegebene Bedeutung hat,
wenn man Phenol-Derivate der Formel (VIII)

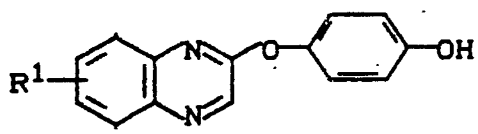

(VIII)

in welcher
$R^1$ die oben angegebene Bedeutung hat,
mit den S-Enantiomeren bzw. R-Enantiomeren von Propionsäureester der Formel (IX)

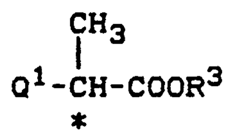

(IX)

in welcher
$Q^1$ für Chlor, Brom, Tosylat oder Mesylat steht und
$R^3$ für Methyl oder Ethyl steht,
gegebenenfalls in Gegenwart eines Säureakzeptors, wie z. B. Kaliumcarbonat, und gegebenenfalls in Gegenwart eines Verdünnungsmittels, wie z. B. Acetonitril, bei Temperaturen zwischen 0 °C und 120 °C umsetzt und die dabei entstehenden Ester der Formel (X)

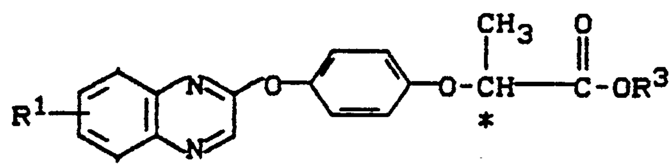

(X)

in welcher
$R^1$ und $R^3$ die oben angegebenen Bedeutungen haben,
mit starken Basen, wie z. B. Natriumhydroxid, in Gegenwart eines Verdünnungsmittels, wie z. B. Methanol, Ethanol, Benzol, Toluol oder Xylol, gegebenenfalls im Gemisch mit Wasser bei Temperaturen zwischen 20 °C und 140 °C verseift und anschließend mit einer Säure, wie z. B. Salzsäure, ansäuert.

In der ersten Stufe dieser Umsetzung findet am asymmetrischen Kohlenstoffatom der Propionsäure-Einheit eine Walden'sche Umkehr statt. Dieses hat zur Folge, daß durch Umsetzung von Phenol-Derivaten der Formel (VIII) mit S-Enantiomeren der Propionsäureester der Formel (IX) die R-Enantiomeren der Ester der Formel (X) entstehen. Andererseits bilden sich durch Umsetzung von Phenol-Derivaten der Formel (VIII) mit R-Enantiomeren der Propionsäureester der Formel (IX) die S-Enantiomeren der Ester der Formel (X).

Die bei dem erfindungsgemäßen Verfahren (a) weiterhin als Ausgangsstoffe benötigten Silylchloride sind durch die Formel (III) definiert. In dieser Formel hat $R^2$ vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Silylchloride der Formel (III) sind bekannt oder lassen sich in einfacher Weise nach bekannten Verfahren herstellen.

Die bei dem erfindungsgemäßen Verfahren (b) als Ausgangsstoffe benötigten Chinoxalinyloxy-phenoxypropionsäurechloride sind durch die Formel (IV) definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Verbindungen der Formel (IV) sind bekannt oder lassen sich nach bekannten Verfahren in einfacher Weise herstellen (vergl. DE-OS 30 04 770). So lassen sich die Chinoxalinyloxy-phenoxypropionsäurechloride der Formel (IV) aus den entsprechenden Propionsäure-Derivaten der Formel (II) durch Chlorierung, z. B. mit Thionylchlorid, nach üblichen Methoden herstellen.

Die bei dem erfindungsgemäßen Verfahren (b) weiterhin als Ausgangsstoffe benötigten Verbindungen sind durch die Formel (V) definiert. In dieser Formel haben $R^2$ und Y vorzugsweise diejenigen Bedeutungen, die bereits im Zusammenhang mit der Beschreibung der erfindungsgemäßen Stoffe der Formel (I) vorzugsweise für diese Substituenten genannt wurden.

Die Verbindungen der Formel (V) sind bekannt oder lassen sich nach bekannten Methoden in einfacher Weise herstellen.

Zur Herstellung von R-bzw. S-Enantiomeren der Verbindungen der Formel (I) werden bei der Durchführung des erfindungsgemäßen Verfahrens (b) die R-bzw. S-Enantiomeren der Chinoxalinyloxy-phenoxypropionsäurechloride der Formel (IV) eingesetzt.

Die bei dem erfindungsgemäßen Verfahren (c) als Ausgangsstoffe benötigten Heterocyclen sind durch die Formel (VI) definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Substituenten genannt wurden. Q steht vorzugsweise für Fluor, Chlor, Brom, Methylsulfonyl, Ethylsulfonyl oder Tosyl.

Die Heterocyclen der Formel (VI) sind bekannt (vergl. z. B. Bull. Soc. Chem. France, 1963, 356; Heterocycles 23, 143 (1985); Chem. Lett. 1984, 323).

Die bei dem erfindungsgemäßen Verfahren (c) weiterhin als Ausgangstoffe benötigten Hydrochinon-Derivate sind durch die Formel (VII) definiert. In dieser Formel haben $R^2$ und Y vorzugsweise diejenigen Bedeutungen, die bereits im Zusammenhang mit der Beschreibung der erfindungsgemäßen Stoffe der Formel (I) vorzugsweise für diese Substituenten genannt wurden.

Die Hydrochinon-Derivate der Formel (VII) sind bekannt (vergl. EP-A 095 115 und EP-A 0 175 199).

Zur Herstellung von R-bzw. S-Enantiomeren der substituierten Chinoxalinyloxy-phenoxypropionsäure-Derivate der Formel (I) werden bei der Durchführung des erfindungsgemäßen Verfahrens (c) die R-bzw. S-Enantiomeren der Hydrochinon-Derivate der Formel (VII) eingesetzt.

Die erfindungsgemäßen Verfahren (a), (b) und (c) zur Herstellung der neuen substituierten Chinoxalinyloxy-phenoxypropionsäure-Derivate der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei den erfindungsgemäßen Verfahren (a), (b) und (c) alle üblicherweise für derartige Umsetzungen verwendbaren Säurebindemittel eingesetzt werden. Vorzugsweise in Frage kommen Alkalimetallhydroxide wie z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4 Diazabicyclo-[2,2,2]octan (DABCO).

Die Reaktionstemperaturen können sowohl bei dem erfindungsgemäßen Verfahren (a) als auch bei den Verfahren (b) und (c) in einem größeren Bereich variiert werden. Im allgemeinen arbeitet man bei Temperaturen zwischen -20 °C und +160 °C, vorzugsweise bei Temperaturen zwischen 0 °C und 140 °C.

Die erfindungsgemäßen Verfahren (a), (b) und (c) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b) und (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b) und (c) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe eignen sich zur selektiven Bekämpfung monokotyler Unkräuter in dikotylen Kulturen insbesondere im Nachauflaufverfahren. Die sehr gute Wirkung der erfindungsgemäßen Wirkstoffe zeigt sich insbesondere beim Einsatz gegen perennierende Gräser der Gattungen Agropyron, Cynodon und Lolium und gegen Ausfallgerste.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Also flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Axo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. 3-(3-Trifluormethylphenyl)-1,1-dimethylharnstoff, Ethyl-2-{[(4-Chlor-6-methoxy-2-pyrimidinyl)-aminocarbonyl]-aminosulfonyl}-benzoat, 4-Amino-6-(1,1-dimethylethyl)-3-methylthio-1,2,4-triazin-5(4H)-on, 4-Amino-3-methyl-6-phenyl-1,2,4-triazin-5-(4H)-on, 5-(2-Chlor-4-trifluormethyl-phenoxy)-N-methylsulfonyl-2-nitrobenzamid, (2-Ethoxy-1-methyl-2-oxo-ethyl)-5-(2-chlor-4-trifluormethyl-phenoxy)-2-nitro-benzoat, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3-Isopropyl-2,1,3-benzothiadiazinon-(4)-2,2-dioxid und 2-[4,5-Dihydro-4-methyl-4-(1-methylethyl)-5-oxo-1H-imidazol-2-yl]-4(5)-methyl-benzoesäuremethylester. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Bevorzugt wird nach dem Auflaufen der Pflanzen appliziert.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

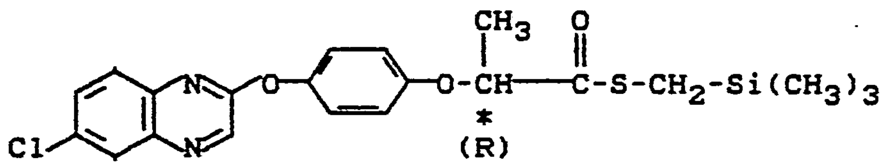

(Verfahren (b))

In einem Gemisch aus 1,56 g (0,013 Mol) (Trimethylsilylmethyl)-mercaptan, 1,6 g (0,016 Mol) Triethylamin und 20 ml Toluol wird bei 0 °C unter Rühren eine Lösung von 4,72 g (0,013 Mol) des R-Enantiomeren des 2-[4-(6-Chlor-chinoxalinyl-2-oxy)-phenoxy]-propionsäurechlorids in 10 ml Toluol eingetropft. Es wird 3 Stunden bei Temperaturen zwischen 0 °C und 5 °C nachgerührt und dann aufgearbeitet, indem man das Reaktionsgemisch mit Wasser verdünnt, das anfallende ölige Produkt mit Toluol extrahiert, die organische Phase schnell mit 5-%iger wäßriger Natronlauge wäscht, dann mit verdünnter Essigsäure und Wasser wäscht und nach dem Trocknen durch Abziehen des Verdünnungsmittels unter vermindertem Druck einengt.

Man erhält auf diese Weise 3,3 g (70 % der Theorie) an dem R-Enantiomeren des 2-[4-(6-Chlor-chinoxalinyl-2-oxy)-phenoxy]-propionsäure-(trimethylsilyl-methyl)-thioesters.

Drehwert: $[\alpha]_D^{24}$ = +20,3° (1-molare Lösung in Chloroform; Küvettenlänge 10 cm)

Beispiel 2

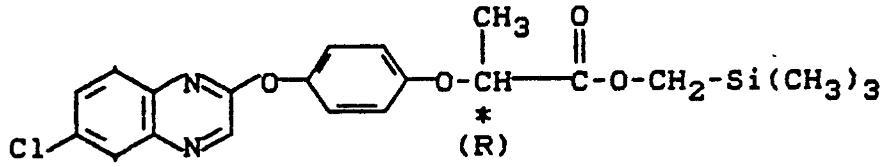

(Verfahren (c))

Ein Gemisch aus 288 g (0,97 Mol) des R-Enantiomeren des 2-(4-Hydroxy-phenoxy)-propionsäure-trimethylsilylmethyl)-esters, 35,7 g Calciumhydroxid und 800 ml Dimethylsulfoxid wird auf 40 °C erhitzt. Bei dieser Temperatur wird eine Lösung von 144 g (0,88 Mol) 2,6-Dichlor-chinoxalin in 200 ml Dimethylsulfoxid unter Rühren langsam zugetropft. Das Reaktionsgemisch wird 2 Stunden bei 40 °C und 16 Stunden bei 20 °C nachgerührt. Danach wird aufgearbeitet, indem man das Reaktionsgemisch in Wasser gießt, das anfallende ölige Produkt mit Toluol extrahiert, die organische Phase nacheinander mit verdünnter wäßriger Natronlauge und Wasser wächst, mit Eisessig schwach sauer stellt, dann trocknet und unter vermindertem Druck einengt.

Man erhält auf diese Weise 270 g (72 % der Theorie) an dem R-Enantiomeren des 2-[4-(6-Chlor-chinoxalinyl-2-oxy)-phenoxy]-propionsäure-(trimethylsilyl-methyl)-esters in kristalliner Form.

Schmelzpunkt: 35 °C

Drehwert: $[\alpha]_D^{24}$ = +31,6° (1-molare Lösung in Chloroform; Küvettenlänge 10 cm)

Analog Beispiel 1 und 2 bzw. den erfindungsgemäßen Verfahren (a), (b) oder (c) können die folgenden Verbindungen der Formel (Ia) hergestellt werden:

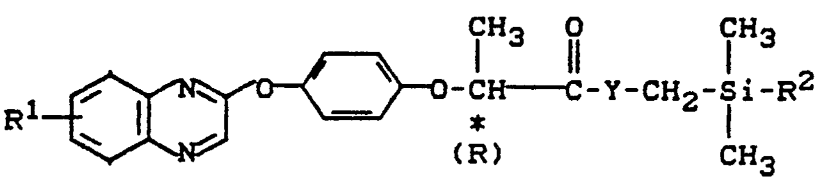

(Ia)

Tabelle 2

| Beisp.-Nr. | $R^1$ | $R^2$ | Y | Physikalische Konstante |
|---|---|---|---|---|
| 3 | 6-Cl | $-C_2H_5$ | O | $n_D^{20}$ = 1,5494 |
| 4 | 6-Cl | $-C_4H_9$-n | O | $n_D^{20}$ = 1,5543 |
| 5 | 6-Cl | $-C_3H_7$-n | O | $n_D^{20}$ = 1,5571 |
| 6 | 6-Cl | $-CH_3$ | NH | |
| 7 | 6-Cl | $-CH_3$ | $NCH_3$ | |
| 8 | 6-Cl | $-C_2H_5$ | NH | |
| 9 | 6-Cl | $-C_3H_7$-n | NH | |
| 10 | 6-Cl | $-C_2H_5$ | $NCH_3$ | |
| 11 | 6-Cl | $-C_3H_7$-n | $NCH_3$ | |
| 12 | 6-Cl | $-C_2H_5$ | S | |
| 13 | 6-Cl | $-C_3H_7$-n | S | |
| 14 | 6-Cl | $-C_4H_9$-n | S | |
| 15 | 7-Cl | $-CH_3$ | S | |
| 16 | 8-Cl | $-CH_3$ | S | |
| 17 | 7-Cl | $-CH_3$ | O | |
| 18 | 8-Cl | $-CH_3$ | O | |

Herstellung von Ausgangssubstanzen

Beispiel (VII-1)

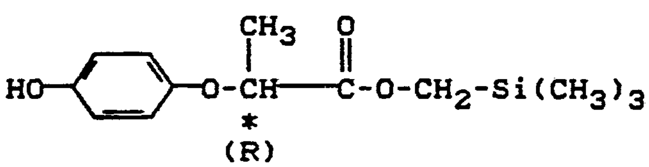

206 g (0,57 Mol) an dem R-Enantiomerem des 2-[4-(Benzyloxyphenoxy)]-propionsäure-(trimethylsilyl)-methylesters werden in 1200 ml Essigester gelöst und in Gegenwart von 40 g Palladium auf Aktivkohle (5-%ig) bei Temperaturen von 40 °C bis 60 °C mit Wasserstoff unter einem Druck von 60 bar hydriert. Anschließend arbeitet man auf, indem man den Katalysator abfiltriert und durch Abziehen des Verdünnungsmittels unter vermindertem Druck einengt.

Man erhält auf diese Weise 150 g (98 % der Theorie) an dem R-Enantiomerem des 2-(4-Hydroxy-phenoxy)-propionsäure-(trimethylsilyl)-methylesters.

Brechungsindex: $n_D^{24}$ = 1,4983

Drehwert: $[\alpha]_D^{24}$ = +6,64° (1-molare Lösung in Chloroform; Küvettenlänge 10 cm)

Nach der im Beispiel (VII-1) beschriebenen Methode lassen sich auch die in der folgenden Tabelle 3 aufgeführten Verbindungen herstellen:

**Tabelle 3**

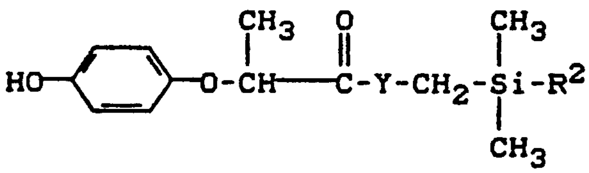

(VII)

| Beispiel Nr. | Y | $R^2$ | Physikalische Konstante |
|---|---|---|---|
| VII-2 | O | $-C_2H_5$ | $n_D^{20}$ : 1,4993 |
| VII-3 | O | $-C_3H_7$-n | $n_D^{20}$ : 1,4945 |
| VII-4 | O | $-C_4H_9$-n | $n_D^{20}$ 1,4972 |
| VII-5 | NH | $-CH_3$ | |
| VII-6 | NH | $-C_2H_5$ | |
| VII-7 | NH | $-C_3H_7$-n | |
| VII-8 | NH | $-C_4H_9$-n | |
| VII-9 | $NCH_3$ | $-CH_3$ | |
| VII-10 | $NCH_3$ | $-C_2H_5$ | |
| VII-11 | $NCH_3$ | $-C_3H_7$-n | |
| VII-12 | $NCH_3$ | $-C_4H_9$-n | |
| VII-13 | S | $-CH_3$ | |
| VII-14 | S | $-C_2H_5$ | |
| VII-15 | S | $-C_3H_7$-n | |
| VII-16 | S | $-C_4H_9$-n | |

Verwendungsbeispiel

In dem nachstehend beschriebenen biologischen Test wird die folgende Verbindung als Vergleichssubstanz eingesetzt:

(A) 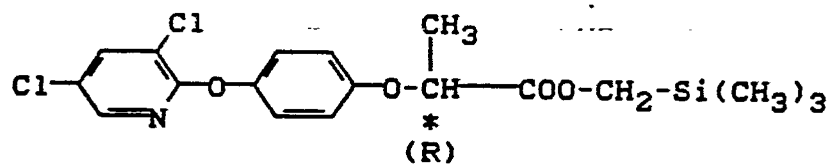

(R)-2-[4-3,5-Dichlorpyridyl-2-oxy)-phenoxy]-propionsäure-(trimethylsilyl)-methylester (bekannt aus EP-A 0 096 354).

Beispiel A

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben zo, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

In diesem Test zeigt z.B. der Wirkstoff gemäß Beispiel (2) bei der Bekämpfung von Ausfallgerste, Cynodon, Lolium und Agropyron in Kulturen wie z. B. Sojabohnen und Baumwolle eine bessere selektive herbizide Wirksamkeit als die Vergleichssubstanz (A).

**Ansprüche**

1. Substituierte Chinoxalinyloxy-phenoxy-propionsäure-Derivate der Formel (I)

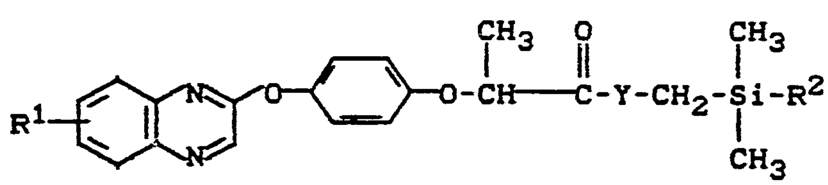 (I)

in welcher
Y für Sauerstoff, Schwefel oder die Gruppierung der Formel NR steht, wobei
R für Wasserstoff oder Alkyl mit 1 bis 6 Kohlenstoffatomen steht,
$R^1$ für Wasserstoff oder Halogen steht und
$R^2$ für Alkyl mit 1 bis 6 Kohlenstoffatomen steht,
sowie deren optisch aktive Verbindungen.

2. Substituierte Chinoxalinyloxy-phenoxypropionsäure-Derivate der Formel (I) gemäß Anspruch 1, in welcher
Y für Sauerstoff, Schwefel oder die Gruppierung der Formel NR, worin

R für Wasserstoff oder Alkyl mit 1 bis 4 Kohlenstoffatomen steht,
$R^1$ für Wasserstoff, Fluor, Chlor oder Brom und
$R^2$ für Alkyl mit 1 bis 4 Kohlenstoffatomen,
sowie deren R-und S-Enantiomere.

3. Substituierte Chinoxalinyloxy-phenoxypropionsäure-Derivate der Formel (I) gemäß Anspruch 1, in welcher
Y für Sauerstoff, Schwefel oder die Gruppierung der Formel NR steht, worin
R für Wasserstoff, Methyl oder Ethyl steht,
$R^1$ für Wasserstoff, Fluor oder Chlor steht und
$R^2$ für Methyl, Ethyl, n-Propyl oder n-Butyl steht.

4. R-Enantiomere von substituierten Chinoxalinyloxyphenoxypropionsäure-Derivaten gemäß Formel (Ia)

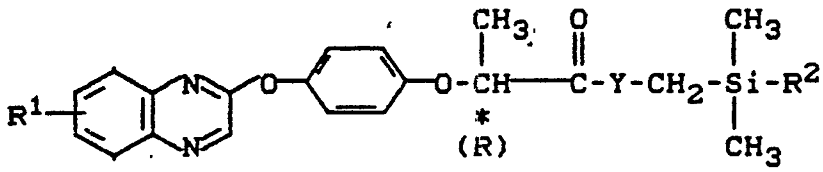

(Ia)

wobei Y, $R^1$ und $R^2$ die in Anspruch 3 angegebene Bedeutung haben.

5. Verfahren zur Herstellung von substituierten Chinoxalinyloxy-phenoxy-propionsäure-Derivate der Formel (I)

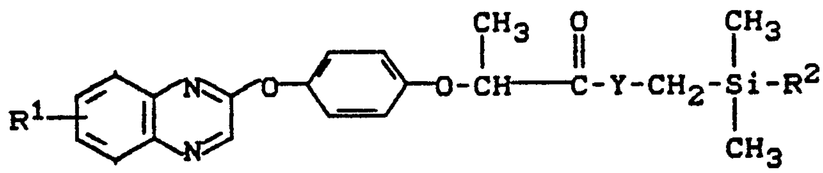

(I)

in welcher
Y für Sauerstoff, Schwefel oder die Gruppierung der Formel NR steht, wobei
R für Wasserstoff oder Alkyl mit 1 bis 6 Kohlenstoffatomen steht,
$R^1$ für Wasserstoff oder Halogen steht und
$R^2$ für Alkyl mit 1 bis 6 Kohlenstoffatomen steht,
dadurch gekennzeichnet, daß man
a) Propionsäure-Derivate der Formel (II)

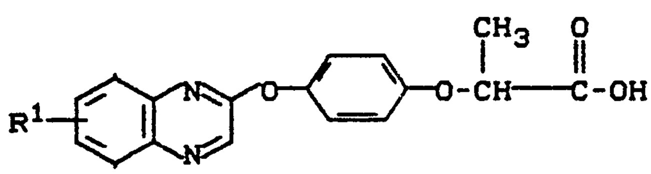

(II)

in welcher
$R^1$ die oben angegebene Bedeutung hat,
mit Silylchloriden der Formel (III)

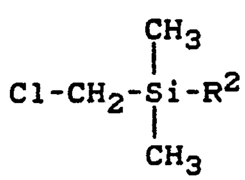

(III)

in welcher
$R^2$ die oben angegebene Bedeutung hat,

gegebenenfalls in Gegenwart eines Säurebindemittels sowie gegebenenfalls in Gegenwart eines Verdünnungsmittels umsetzt,
oder daß man
b) Chinoxalinyloxy-phenoxypropionsäurechloride der Formel (IV)

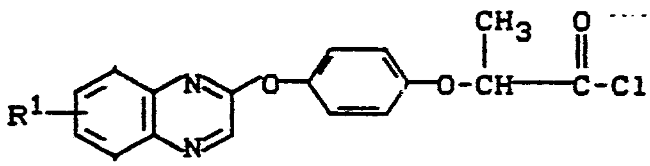

(IV)

in welcher
$R^1$ die oben angegebene Bedeutung hat,
mit Verbindungen der Formel (V)

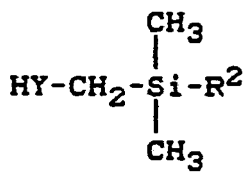

(V)

in welcher
$R^2$ und Y die oben angegebenen Bedeutungen haben,
gegebenenfalls in Gegenwart eines Säurebindemittels sowie gegebenenfalls in Gegenwart eines Verdünnungsmittels umsetzt. oder daß man
c) Heterocyclen der Formel (VI)

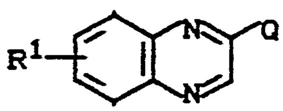

(VI)

in welcher
$R^1$ die oben angegebene Bedeutung hat und
Q für Halogen, Methylsulfonyl, Ethylsulfonyl oder Tosyl steht,
mit Hydrochinon-Derivaten der Formel (VII)

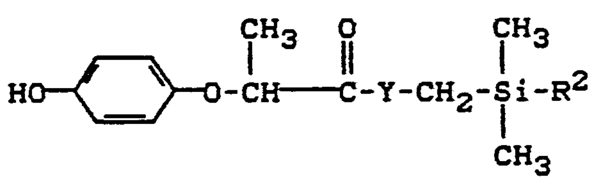

(VII)

in welcher
$R^2$ und Y die oben angegebenen Bedeutungen haben,
gegebenenfalls in Gegenwart eines Säurebindemittels sowie gegebenenfalls in Gegenwart eines Verdünnungsmittels umsetzt.

6. Herbizide Mittel, gekennzeichnet durch einen Gehalt an mindestens einem substituierten Chinoxalinyloxyphenoxypropionsäure-Derivat der Formel (I) gemäß den Ansprüchen 1 bis 5.

7. Verfahren zur Bekämpfung von Unkräutern, dadurch gekennzeichnet, daß man substituierte Chinoxalinyloxy-phenoxypropionsäure-Derivate der Formel (I) gemäß den Ansprüchen 1 bis 5 auf die Unkräuter und/oder ihren Lebensraum einwirken läßt.

8. Verwendung von substituierten Chinoxalinyloxy-phenoxypropionsäure-Derivaten der Formel (I) gemäß den Ansprüchen 1 bis 5 zur Bekämpfung von Unkräutern.

9. Verfahren zur Herstellung von herbiziden Mitteln, dadurch gekennzeichnet, daß man substituierte Chinoxalinyloxy-phenoxypropionsäure-Derivate der Formel (I) gemäß den Ansprüchen 1 bis 5 mit Streckmitteln und/oder oberflächenaktiven Substanzen mischt.

10. Ein substituierte Chinoxalinyl-phenoxypropionsäure-Derivat der Formel

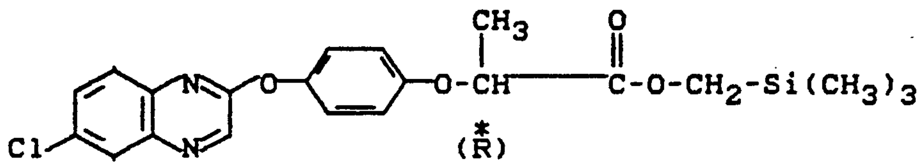

Europäisches Patentamt

# EUROPÄISCHER RECHERCHENBERICHT

Nummer der Anmeldung

EP 87 10 5460

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 171 724 (HOECHST AG)<br>* Seiten 1-4; Beispiele; Patentansprüche *<br>--- | 1-10 | C 07 F 7/08<br>A 01 N 55/00 |
| D,A | EP-A-0 175 199 (BAYER AG)<br>* Patentansprüche *<br>----- | 1-10 | |

RECHERCHIERTE SACHGEBIETE (Int. Cl.4)

C 07 F 7/00
A 01 N 55/00

Der vorliegende Recherchenbericht wurde für alle Patentansprüche erstellt.

| Recherchenort | Abschlußdatum der Recherche | Prüfer |
|---|---|---|
| DEN HAAG | 05-08-1987 | PAUWELS G.R.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82